# EUROPEAN PATENT SPECIFICATION

(11) **EP 1 716 417 B1**
(45) Date of publication and mention of the grant of the patent: **05.03.2008**
(21) Application number: 05718967.2
(22) Date of filing: 21.02.2005
(51) Int. Cl.: G01N 33/539, G01N 33/532, G01N 33/549

(54) **A DIAGNOSTIC KIT FOR DETECTING PULMONARY AND EXTRA PULMONARY TB**
DIAGNOSTISCHER KIT ZUM NACHWEIS PULMONALER UND EXTRAPULMONALER TB
KIT DIAGNOSTIQUE DESTINE A LA DETECTION DE LA TUBERCULOSE PULMONAIRE OU EXTRAPULMONAIRE

(30) Priority: 19.02.2004 IN DE02262004
(43) Date of publication of application: 02.11.2006
(73) Proprietor: DEPARTMENT OF BIOTECHNOLOGY, MINISTRY OF SCIENCE AND TECHNOLOGY, GOVERNMENT OF INDIA, New Delhi 110 003 (IN); MADHAV INSTITUTE OF TECHNOLOGY AND SCIENCE, Gwalior (IN)
(72) Inventor: BISEN, Prakash Sing, Dep. of Biotechnology, New Delhi 110 003 (IN); TIWARY, Ram, Pramod, Gwalior (IN)
(74) Representative: Green, Mark Charles
(86) International application number: PCT/IN2005/000063
(87) International publication number: WO 2005/080987

(56) References cited:
- US-A- 4 605 630
- US-A- 5 620 903

## Description

### FIELD OF THE INVENTION:

This invention relates to a diagnostic kit for detecting pulmonary & extra pulmonary tuberculosis.

### BACKGROUND OF THE INVENTION:

The conventional methods for detecting tuberculosis is time consuming & labour consuming AFB staining is considered to be insensitive (requiring 10,000 organism/ml of sputum for smear positive result with 100X microscope, refer Todar's Text Book of Bacteriology Online). ELISA-KP 90 is also known to be of low sensitivity and specificity and specificity (cut-off value >1.0 +ve, and <0.8 -ve test result) and requires sophisticated infrastructure as also the hypersensitivity based Tuberculin Skin Test (Montaux test) which lacks sensitivity and specificity in BCG vaccinated patient (Constantin P.et.al., Inf & Imm 1998;66). In the same way MYCODOT is inconvenient for HIV correlated individuals (refer G.R.Somi et.al., Int J Tubercle and Lung Disease, 19999, vol 3) and Bactec-460 and Roche molecular system PCR based product) are though sensitive requires very costly infrastructure and technical expertise.

### OBJECTS OF THE INVENTION:

An object of this invention is to propose a diagnostic kit for detecting tuberculosis.

A further object of this invention is to propose a diagnostic kit which is economical and easy to handle.

A stilt further object of this invention is to propose a diagnostic kit based on liposome agglutination.

Another object of this invention is to propose a diagnostic kit which is sensitive and specific as it is based on a specific antigenic, antibody reaction.

Still another object of this invention is to propose a diagnostic kit which helps in fast detection of tuberculosis.

### BRIEF DESCRIPTION OF THE INVENTION

According to this invention there is provided a diagnostic kit for detecting pulmonary & extra pulmonary tuberculosis comprising a test card "TB Screen" coated with a hydrophobic material, antigen suspension, positive and Negative control.

In accordance to this invention there is provided a method of detecting tuberculosis using the kit comprising applying positive control, negative control & test sample each in circular motion on the test card coated with hydrophobic material adding said antigen suspension to each of the positive, negative & test sample to interpart the results, clumping of specific antigen and anti body as dark blue agglutination was observed in positive control and the test sample which contain the active tuberculosis infection*.*

### DETAILED DESCRIPTION OF THE INVENTION

The Mycobacteriun tuberculosis H₃₇Rv (ATCC-27294) strains was grown on Sautons media till late log phase (2-3 month) and cells were harvested by centrifugation (5000,-10,000 g for 10-20 min) at 4-10 C, the pellet was washed with PBS (pH 7.2-7.5), resuspended with TEN buffer PH 8.0-8.5 (10mM Tris HC1, 1mM EDTA, 100mM NaCl) and heat inactivated at 70-80C (waterbath) for 30-45 min. followed by sonication. The glycolipid antigens were extracted according to the procedure mentioned in the literature (Reggiardo et al., 1974; Bisen P.S. et al., 2003) with the slightly modification in the procedure. The lypolized Mycobacterial powder (10-15g) was taken into a glass reagent bottle and to it 100-150ml of chloroform and methanol mixture (2:1) was added. This was stirred at room temperature for 50-60 min. and filtered through whatman filter paper No 1. The 1/5 volume of 0.7%KCl (20.0 ml) was added to the filtrate and was shaken for 5-6 times. The suspension was transferred to a separating funnel and kept at 2-8°C for overnight till two distinct layers were separated. The lower organic phase was washed with 1/5 volume of washing solvent (C:M:W:3:48:47) in similar manner by keeping at 2-8°C for overnight. The upper aqueous phase was removed and tower organic phase was retained after filtering. The organic phase was dried by evaporating the solvent in rotatory solvent evaporator at 40-50°C. The moisture was removed by flushing the dried mixture with nitrogen gas. Neutral lipids were removed from the dried mixture by adding 300-500 ml of chilled acetone vortexig it for 10-20 min and filtering it through whatman No.1. This step was repeated fill the lipids in the flask became whitish or colorless. This was filtered through whatman No.1 and the filtrate was discarded. The lipids present on the filter paper were dissolved with C:M (2:1) and transferred to the R.B flask. Solvent was rotary evaporated under reduced pressure at 40-50°C, The crude preparation was reconstituted in 10-15 ml of C:M (2:1) and stored at -20°C for further use.

### Purification of Antigen(s):

The Silica gel H activated at 100-110°C for 1-1.30 hrs. (Hot air oven) was packed with glass column (2.5X30 cm) with manual tapping and known quantity of crude material (1g/5 ml, stock) was loaded on either side of the column. The column was run ascending on chromatographic jar (4.5X25cm) with purification solvent, 150-200ml (mobile phase) in a ratio of 65:25:4 (C:M: W)^{7,8} at room temperature to run the column till other it reached the end. The column was removed from the chromatographic jar and placed on fume hood to evaporate the solvent from the column. The 1 cm length of each fraction was carefully scrapped using clean rod so as to get the separate molecules which were adsorbed with the silica gel depending upon the mobility and Retardation Factor (RF) value (46.6, 53.4, 58.3, 67.2 and 72.4%) of individual molecule. The individual fraction was collected into clean dry glass test tubes which were labeled with respective fraction number. Ten ml of extraction solvent (mixture of chloroform: Methanol 2:1) was added to each test tubes and kept at room temperature for 30-40min. The purity of eluted material was analyzed by TLC and the selected fraction were further filtered through Whatman filter paper No.1 to remove the silica gel from the samples. The pure fractions were pooled and these were characterized by conventional methods (immuno-staining on TLC, ELISA and by Liposome).

### Method for the construction of Liposomal antigens:

Liposome was prepared as described previously (Bangham A.D et. al.1965) with minor modification in the procedure, in brief Phophotidylcholine 100-150mg; cholesterol, 450-500 mg (Sigma, USA); antigenic suspension (Cocktail)10-20 mg; and dye50-100µl (1.0 % sudan black B in Chloroform) were taken in a predried round bottom flask. Solvent was evaporated by rotatory vacuum evaporator under reduced pressure. The dried contents were dissolved in 40-50 ml of absolute alcohol (99.9%Hyman, Germany) and were kept at 4°10°C for 1-1.30 hrs. Sucrose solution (4-8 ml; 150Mm) was taken in a polypropylene centrifuge tube (capacity 35 ml) and to that 4-5 ml of pre-prepared alcoholic antigen suspension was gently added while vortexing. The tubes were kept over night at 4°C-10C for liposome swelling, vortexed with 10-15 ml of PBS (pH 6.5) buffer and centrifuged at 10, 000g for 10-20 min (Beckman, USA). The supernatant was discarded and the pellet was resuspended with 20-30 ml of B2 buffer, pH 7.2 (NaH₂PO₄.2H₂O, 10mM; KH₂PO₄, 10mM; EDTA, 10mM; Choline Chloride, 10% and Thiomersol, 0.1%). This was stored at 4°C-10C for further use and utilized as antigen reagent for the kit.

### Preparation of Phosphatidylcholine:

Phosphatidylcholine (PC) was prepared in house to reduce the cost of the test. Those skilled in the art are aware of purification of PC from egg yolk and it's final estimation (Sunamoto, J.et al 1978). In brief, 25 eggs were taken and albumin portions were removed. The collected yolk was extracted with 750 ml of Chloroform: Methanol (2:1) by stirring for half an hour. Filtered through Whatmann no. 1. The filterate was deproteinised with one fifth volume of 0.7% of KC1. To the organic layer so obtained, washing was performed with 3:48:47 of Chloroform: methanol: water. Moisture was removed by using benzene. Solvent was evaporated with the aid of rotary vacuum evaporated with the aid of rotary vacuum evaporator and a dried film of lipid was obtained. Neutral lipids were removed as described above with acetone. Empty weight of round bottom flask was taken (Wa). Flask was weighed along with the dried lipid film. 37.5 g of crude lipid was isolated. The crude product was further purified by silica gel H chromatography and purified PC was characterized by Thin layer chromatography and PC estimation was performed as known in the prior art (Sunamoto, J.et al 1978).

### Preparation of positive control for test:

1 mg of Mycobacterium tuberculosis pellet was taken after centrifugation of mycobacterial growth in Sauton's medium. The pellet was washed twice with 1X PBS to get rid of media remnants. The pellet was then suspended in 4 ml of 1X Phosphate buffered saline. 4-8 acid washed beads of 5 mm diameter was added to the above. The sample was vigorously shaken for 10 mm on a vertex. The suspension obtained was mixed with an equal volume of Freunds Incomplete Adjuvant. The mixture was squeezed through 22g needle repeatedly till it reaches a desired level. 100 ul of suspension was inoculated to a young rabbit of 2-8 months. A number of rabbits were inoculated in the same manner.

Rabbits were bled after one month and serum was obtained. The reactivity of serum was checked with the antigen suspension as described in the test procedure given in next paragraph. The reactivity titer was checked. A booster administration of the antigen (50 ul) was again repeated. An enhanced titer of about 1:64 to 1:128 was obtained after about seven days of booster dosage. Best reactivity titer was obtained. The serum was diluted to optimum reactivity titer in 1X PBS and 0.1% azide was added as preservative so as to contain any contamination. The stock was frozen till used/dispensed in vials.

The 4-6 month old Rabbits were immunized with the above antigens and bled periodically and used for positive control to be provided with the kits, where as normal young Rabbit were used for Negative control.

### Method of testing:

All the components such as positive control, negative control, antigen suspension and sample to be tested were brought to room temperature before performing the experiments. Positive control, negative control and test sample (25µl each) were added in circular motion, as demonstrated on the test card. The above samples were spread by using separate sticks in round conjugation, 25 µl of liposome antigen was added to it and the card was gently swirled for 4 min.

Freshly procured or frozen test serum samples (25 µl) were spread evenly inside the circular zone of hydrophobic material coated plastic slide. For convenience, zone 1 and 2 were spread with the positive (anti-rabbit serum) and negative control (normal rabbit serum) and negative control (normal rabbit serum) respectively, to interpret the results. The antigenic suspension (25 µl) was added to each circular zone including zone 1 and 2 and the card was manually swirled for 4 min. The clumping of specific antigen and antibody as dark blue agglutination were observed in positive control as well as In those samples which contain antibodies against mycobacterial glycolipid with active tuberculosis infection. No clumping on the card whereas, indicated a negative result. The peripheral drying on the circular zone indicated indiscriminate results, which require further confirmation within 15-30 days, as these samples contained undetectable level of antigen concentration in the specimens.

### EXAMPLE 1

### SERA:-

Patient sera from outdoor patient departments (OPD) from different hospitals of India were enrolled in the present study to cover maximum population diversity. The patients were diagnosed on the basis of clinical and radiological evaluation as well as smear staining and sputum culture of samples. None of the patients was completely treated. Both extrapulmonary as well as pulmonary tuberculosis sera were included in the study. A total of three hundred and twenty four (324) tuberculosis sera were studied.

Sera from healthy individuals without any clinical symptoms of TB were included as negative controls to evaluate specificity criterion of the test. Most of these were obtained from BCG vaccinated subjects. The non-TB sera generally belonged either to healthy individuals or to patients suffering from a variety of diseases other than tuberculosis. The sera were stored frozen and were used within 1 year from the time, they were taken. Also five hundred and eleven (511) tuberculosis negative sera were included in this testing. The details of criteria used in seiection of sera is as follows:
- Smear Negative, Culture Positive pulmonary cases ---52
- Smear Positive, Culture Positive, pulmonary cases ---180
- Extrapulmonary, Culture Positive cases --- 35
- Relapse pulmonary cases ---57
- Drug treated, clinically negative cases ---60
- Healthy household contacts --- 50
- BCG vaccinated children ---15
- Hepatitis B positive samples ---15
- Sera from common infections other than TB ---27
- Normal human sera ---344

### EXAMPLE 2

### IN HOUSE EVALUATIONS:-

An overall sensitivity of 98.63% was obtained using a panoply of three hundred and twenty four tuberculosis sera, out of which 20 sera showed indiscriminate results. Indiscriminate sera were not included in the sensitivity and specificity calculations, as per method adopted by WHO. An overall specificity of 98.78% was obtained using five hundred and eleven non-tuberculosis sera.

Sera from 15 children who were recently immunized with BCG were tested for any cross-reactivity of the test with vaccination. None of the sera yielded positive results, thereby indicating the suitability of the test in BCG vaccinated populations such as india and others.

15 cases of Hepatitis B positive samples were evaluated for cross reactivity. There was no reaction in any of the sera tested. 4 Hepatitis B sera were tested with the kit at Hopkins Research Institute, Bombay with nil reactivity (not included in inhouse study table).

Out of 27 sera from other common infections, 25 showed clear negative and rest two showed indiscriminate results. There was a need to chase these subjects for progression to tuberculosis, but unfortunately, it could not be done. Indeterminate results were omitted from specificity and sensitivity calculations.

Results of inhouse studies are tabulated as follows:

**Table 1**

| STUDIES PERFORMED ON TUBERCULOSIS POPULATION | |
|---|---|
| SERA DETAILS | RESULTS |
| • Number of tuberculosis sera tested=324 | positive=300 |
| | negative=4 |
| | indeterminate=20 |
| | SENSITIVITY= 98.68% |
| Smear Negative, Culture Positive pulmonary cases | positive=47 |
| ---52 | negative= 2 |
| | indeterminate=3 |
| Smear Positive, Culture Positive, pulmonary cases | positive =175 |
| --- ---180 | negative = --- |
| | indeterminate =5 |
| Extrapulmonary cases, Culture Positive | positive=28 |
| ---35 | negative =2 |
| | indeterminate=5 |
| Relapse pulmonary cases | positive=50 |
| ---57 | negative= --- |
| | indeterminate = 7 |

| | |
|---|---|
| **indiscriminate samples were not included in Sensitivity & specificity calculations as per WHO methodology | |

**Table 2**

| STUDIES PERFORMED ON NON-TUBERCULOUS SERA | |
|---|---|
| • Number of non-TB sera tested =511 | positive =488 |
| | negative=6 |
| | indeterminate=17** |
| | SPECIFICITY=98.78% |
| 5. Drug treated, clinically negative cases | positive= --- |
| ---60 | negative=51 |
| | indeterminate=9 |
| 6. Healthy household contacts ---50 | positive= --- |
| | negative=50 |
| | indeterminate= --- |
| 7. BCG vaccinated children ---15 | positive= --- |
| | negative=15 |
| | indeterminate= -- |
| 8. Hepatitis B positive samples ---15 | positive= --- |
| | negative= 15--- |
| | indeterminate = |
| 9. Sera from common infections (Not TB) | positive = -- |
| ---27 | negative=25 |
| | indeterminate = 2 |
| 10. Normal human sera ---344 | positive = 6 |
| | negative = 332 |
| | indeterminate = 6 |

| | |
|---|---|
| **indiscriminate samples were not included in Sensitivity & specificity calculations as per WHO methodology | |

Excellent results were obtained when using fresh sera from subjects under investigation. Frozen sera can be tested after thawing, but repeated freeze thawing of samples might affect the outcome.

## Claims

1. A diagnostic kit for detecting pulmonary and extra pulmonary tuberculosis comprising a test card marked "TB Screen" coated with a hydrophobic material, Mycobacterium tuberculosis antigen suspension presented in a liposome, wherein the antigen preparation is a glycolipid preparation, and a positive and negative control.

2. A kit as claimed in claim 1, wherein said test card is preferably a plastic slide.

3. A kit as claimed in claim 1, wherein said negative control is prepared from the blood of normal young rabbit.

4. A kit as claimed in claim 1, wherein said positive control is prepared from 4 to 6 month old rabbit which were immunized with the Mycobacterium antigens and bled periodically.

5. A method of detecting tuberculosis using the kit as claimed in claim 1, comprising the steps of:
applying positive control, negative control and test sample each in a circular motion on the test card which is coated with hydrophobic material,
adding said antigen suspension to each of the positive, negative and test sample to interpret the results,
observing clumping of specific antigen and antibody as dark blue agglutination in the positive control and the test sample which contain the active tuberculosis infection.

6. The method as claimed in claim 5, wherein the lipid antigens for positive control are prepared comprising the steps of:
growing Mycobacterium tuberculosis H 37Rᵥ(ATTC-27294) strain on Sautons media,
harvesting the cell in the media by centrifugation at 4° to 10°C,
subjecting the said cells to the step of sonication,
extracting the antigens from the said cells,
adding chloroform and methanol mixture (2:1) to the said antigens with stirring at room temperature,
subjecting the mixture to the step of filtration,
the suspension thus obtained is transferred into a separating funnel and kept for overnight till two distinct layers were separated, upper aqueous phase was removed and the lower organic phase retained after filtration, organic phase was dried by evaporating the solvent to obtain the lipids and subjecting the said lipid to the further step of purification.

7. A method as claimed in claim 5, wherein said antigen suspension is prepared comprising the steps of:
adding phophotidylcholine, cholesterol, lipid antigens and dye in a flask and evaporating the solvent in a vacuum evaporator,
dissolving the dried contents thus obtained in absolute alcohol and kept at 4° to 10°C for 1 to 2 hrs to produce the antigen suspension,
adding the said antigen solution to sucrose solution with continuous stirring and the said suspension was kept at 2-8°C overnight,
subjecting the said suspension to the step of centrifugation, supernatant was discarded,
suspending the pellet into the buffer and string the same at 4° to 10°C.

8. The method as claimed in claim 5, wherein the said lipid (antigen) is further purified using column chromatography.

9. The method as claimed in claim 7, wherein the said buffer comprises NaH₂PO₄2H₂O, KH₂PO₄, EDTA, Choline Chloride and Thiomersol.

10. The method as claimed in claim 7, wherein the said dye is sudan Black in chloroform.

## Patentansprüche

1. Diagnostik-Kit zum Nachweis der pulmonalen und extrapulmonalen Tuberkulose, umfassend eine als "TB-Screen" bezeichnete Testkarte, beschichtet mit einem hydrophoben Material, einer in einem Liposom vorliegenden Mycobacterium tuberculosis-Antigensuspension, worin die Antigenpräparation eine Glycolipidpräparation darstellt, und einer positiven und negativen Kontrolle.

2. Kit nach Anspruch 1, worin die Testkarte bevorzugt einen Kunststoffobjektträger darstellt.

3. Kit nach Anspruch 1, worin die negative Kontrolle aus dem Blut von gesunden jungen Kaninchen hergestellt ist.

4. Kit nach Anspruch 1, worin die positive Kontrolle aus 4 bis 6 Monate alten Kaninchen, die mit den Mycobacterium-Antigenen immunisiert und periodisch geblutet wurden, hergestellt ist.

5. Verfahren zum Nachweis von Tuberkulose unter Verwendung des Kits nach Anspruch 1, umfassend die folgenden Schritte:
Aufbringen der positiven Kontrolle, negativen Kontrolle und der Testprobe jeweils in einer kreisförmigen Bewegung auf die mit hydrophobem Material beschichtete Testkarte,
Zufügen der Antigensuspension zu jeweils der positiven Probe, negativen Probe und Testprobe zum Interpretieren der Ergebnisse,
Beobachten der Verklumpung von spezifischem Antigen und Antikörper als dunkelblaue Agglutination in der positiven Kontrolle und der Testprobe, welche die aktive Tuberkuloseinfektion enthalten.

6. Verfahren nach Anspruch 5, worin die Lipidantigene für die positive Kontrolle hergestellt werden, umfassend die folgenden Schritte:
Züchten des H 37Rᵥ(ATTC-27294)-Stamms von Mycobacterium tuberculosis auf Sauton-Medium,
Ernten der Zellen im Medium durch Zentrifugation bei 4 ° bis 10 °C,
Aussetzen der Zellen gegenüber dem Beschallungsschritt,
Extrahieren der Antigene aus den Zellen,
Zufügen eines Gemischs aus Chloroform und Methanol (2:1) zu den Antigenen unter Rühren bei Raumtemperatur,
Aussetzen des Gemischs gegenüber dem Filtrationsschritt;
die auf diese Weise erhaltene Suspension wird in einen Trenntrichter überführt und dort über Nacht zurückgehalten, bis sie in zwei distinkte Schichten aufgetrennt war, die obere wässrige Phase wurde entfernt und die untere organische Phase nach dem Filtrieren zurückbehalten, die organische Phase wurde zum Erhalt der Lipide durch Verdampfung des Lösungsmittels getrocknet und die Lipide einem weiteren Reinigungsschritt ausgesetzt.

7. Verfahren nach Anspruch 5, worin die Antigensuspension hergestellt wird, umfassend die folgenden Schritte:
Zufügen von Phosphatidylcholin, Cholesterol, Lipidantigenen und Farbstoff in einen Kolben und Verdampfen des Lösungsmittels in einem Vakuumverdampfer,
Auflösen des auf diese Weise erhaltenen getrockneten Inhalts in absolutem Alkohol und ein- bis zweistündiges Stehenlassen bei 4 °C bis 10 °C zum Herstellen der Antigensuspension,
Zufügen der Antigenlösung zur Saccharoselösung unter kontinuierlichem Rühren und Aufbewahren der Suspension über Nacht bei 2 bis 8 °C,
Aussetzen der Suspension gegenüber dem Zentrifugationsschritt, der Überstand wurde verworfen,
Suspendieren des Pellets im Puffer und Lagern desselben bei 4 °C bis 10 °C.

8. Verfahren nach Anspruch 5, worin das Lipid (Antigen) mithilfe der Säulenchromatographie weiter gereinigt wird.

9. Verfahren nach Anspruch 7, worin der Puffer NaH₂PO₄·2H₂O, KH₂PO₄, EDTA, Cholinchlorid und Thiomersal umfasst.

10. Verfahren nach Anspruch 7, worin der Farbstoff Sudanschwarz in Chloroform darstellt.

## Revendications

1. Kit de diagnostic en vue de la détection de la tuberculose pulmonaire et extra pulmonaire, comprenant une carte d'essai marquée "Écran TB" revêtue d'un matériau hydrophobe, une suspension antigène de Mycobacterium tuberculosis présentée dans un liposome, dans lequel la préparation antigène est une préparation de glycolipides, et un contrôle positif et négatif.

2. Kit selon la revendication 1, dans lequel ladite carte d'essai est, de préférence, une micro plaquette en matière plastique.

3. Kit selon la revendication 1, dans lequel ledit contrôle négatif est préparé à partir du sang d'un jeune lapin normal.

4. Kit selon la revendication 1, dans lequel ledit contrôle positif est préparé à partir de lapins âgés de 4 à 6 mois qui ont été immunisés grâce aux antigènes Mycobacterium et qui ont été périodiquement saignés.

5. Procédé de détection de la tuberculose par utilisation du kit selon la revendication 1, comprenant les étapes :
d'application du contrôle positif, du contrôle négatif et de l'échantillon d'essai, chacun étant mis en mouvement circulaire sur la carte d'essai, qui est revêtue d'un matériau hydrophobe,
d'addition de ladite suspension antigène à chacune des entités contrôle positif, contrôle négatif et de l'échantillon d'essai pour interpréter les résultats,
d'observation de l'agglutination de l'antigène spécifique et de l'anticorps en tant que produit d'agglutination de couleur bleu sombre dans le contrôle positif et dans l'échantillon d'essai, qui contiennent l'infection de tuberculose active.

6. Procédé selon la revendication 5, dans lequel les antigènes de lipides pour le contrôle positif sont préparés, lequel procédé comprend les étapes :
de culture de l'espèce de Mycobacterium tuberculosis H 37Rᵥ(ATTC-27294) sur un milieu Sautons,
de récolte de la cellule dans le milieu par centrifugation à la température de 4°C à 10°C,
d'exposition desdites cellules à l'étape de sonication,
d'extraction des antigènes desdites cellules,
d'addition du mélange de chloroforme et de méthanol (2:1) auxdits antigènes avec agitation à la température ambiante,
d'exposition du mélange à l'étape de filtration,
la suspension ainsi obtenue étant transférée dans un entonnoir de séparation et y étant maintenue pendant la nuit jusqu'à séparation de deux couches distinctes, la couche aqueuse supérieure étant éliminée et la couche organique inférieure étant retenue après filtration, la phase organique étant séchée par évaporation du solvant pour obtenir les lipides et soumettre lesdits lipides à l'étape supplémentaire de purification.

7. Procédé selon la revendication 5, dans lequel ladite suspension d'antigènes est préparée, comprenant les étapes :
d'addition de phosphotidylcholine, de cholestérol, d'antigènes de lipides et de colorant à une flasque et d'évaporation du solvant dans un évaporateur à vide,
de dissolution du contenu desséché ainsi obtenu dans de l'alcool absolu et maintenu à la température de 4°C à 10°C pendant une durée de 1 à 2 heures pour produire la suspension d'antigènes,
d'addition de ladite solution d'antigènes à la solution de sucrose sous agitation continue et en maintenant ladite suspension à la température de 2-8°C pendant la nuit,
d'exposition de ladite suspension à l'étape de centrifugation, le surnageant étant mis au rebut,
de suspension des pastilles dans le tampon et de maintien de celle-ci à la température de 4°C à 10°C.

8. Procédé selon la revendication 5, dans lequel ledit (antigène) lipide est encore purifié par utilisation du procédé de chromatographie sur colonne.

9. Procédé selon la revendication 7, dans lequel ledit tampon comprend du NaH₂PO₄2H₂O, du KH₂PO₄, de l'EDTA, du chlorure de choline et du Thiomersol.

10. Procédé selon la revendication 7, dans lequel ledit colorant est du noir Soudan dans le chloroforme.
